# NEUE EUROPÄISCHE PATENTSCHRIFT

(11) **EP 0 049 740 B2**
(45) Veröffentlichungstag und Bekanntmachung des Hinweises auf die Entscheidung über den Einspruch: **08.02.1995**
(45) Hinweis auf die Patenterteilung: 22.02.1984
(21) Anmeldenummer: 81105361.0
(22) Anmeldetag: 09.07.1981
(51) Int. Cl.: C07C 409/34, C07C 407/00

(54) **Verfahren zur kontinuierlichen Herstellung von Dialkylperoxydicarbonaten**
Process for the continuous preparation of dialkylperoxydicarbonates
Procédé de préparation continue de dialcoyle peroxydicarbonates

(30) Priorität: 09.10.1980 DE 3038164
(43) Veröffentlichungstag der Anmeldung: 21.04.1982
(73) Patentinhaber: Peroxid-Chemie GmbH, 82049 Pullach (DE)
(72) Erfinder: Appel, Hans, D-8023 Höllriegelskreuth (DE); Brossmann, Gottfried, D-8023 Höllriegelskreuth (DE)
(74) Vertreter: Weickmann, Heinrich, Dipl.-Ing.

(56) Entgegenhaltungen:
- DE-B- 1 265 154
- DE-B- 1 643 599
- DE-B- 2 334 269
- GB-A- 1 239 088
- US-A- 3 657 311
- US-A- 3 657 312
- US-A- 3 821 273
- US-A- 3 950 375
- Research Disclosure, May 1980; 19302
- Firmenschrift der Fa. PEROXID-CHEMIE GmbH "Organische Peroxide, Allgemeine Information, Halbwertszeiten"

## Beschreibung

Die Erfindung betrifft ein Verfahren zur kontinuierlichen Herstellung von Dialkylperoxydicarbonaten der allgemeinen Formel I 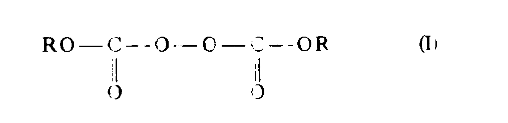
in der R für gleichartige oder verschiedene, geradkettige, cyclische oder verzweigte Alkylgruppen mit 6 bis 18 Kohlenstoffatomen stehen, und insbesondere zur kontinuierlichen Herstellung von Dimyristylperoxydicarbonat und Dicetylperoxydicarbonat, durch kontinuierliches Umsetzen eines Chlorameisensäureesters der allgemeinen Formel II 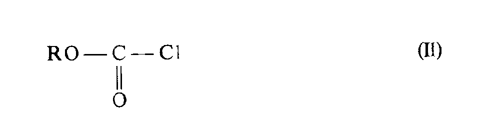
worin R die oben angegebenen Bedeutungen besitzt bzw. für die Myristylgruppe oder die Cetylgruppe steht, in wäßrigem Medium mit Wasserstoffperoxid und einem Alkalimetallhydroxid bei einer Temperatur im Bereich von -10°C bis +50°C in mindestens zwei hintereinander geschalteten Reaktionszonen und kontinuierliches Isolieren des Dialkylperoxydicarbonats aus der lösungsmittelfreien Reaktionsmischung mit Hilfe einer Zentrifuge.

Die kontinuierliche Herstellung von Dialkylperoxydicarbonaten ist bereits seit langem bekannt. So offenbaren die deutschen Auslegeschriften 1 265 154 und 1 259 325 solche Verfahren, bei denen die Umsetzung der Reaktionsteilnehmer, nämlich des Chlorameisensäureesters, des Wasserstoffperoxids und des Alkalihydroxids in wäßrigen, Medium erfolgt und die Reaktionsprodukte in einem organischen Lösungsmittel aufgenommen werden. Diese Verfahren vermögen insbesondere wegen der eingesetzten organischen und insbesondere halogenhaltigen Lösungsmittel nicht zu befriedigen.

Aus der DD-PS 108 272 ist ebenfalls ein Verfahren zur kontinuierlichen Herstellung von Dialkylperoxydicarbonaten durch Umsetzung von Chlorameisensäureestern mit Wasserstoffperoxid in Gegenwart von Alkalimetallhydroxid bekannt, das ebenfalls vorzugsweise in Gegenwart eines reaktionsinerten Lösungsmittels durchgeführt wird.

Schließlich offenbart die US-PS 3 950 375 das eingangs beschriebene lösungsmittelfreie Verfahren zur kontinuierlichen Herstellung von Dialkylperoxydicarbonaten. Hierbei erfolgt die Abtrennung der festen Dialkylperoxydicarbonate von der Reaktionsmischung unter Anwendung einer Flüssigkeits-Feststoff-Zentrifuge.

In DE-B-2 334 269 wird ein Verfahren zur Herstellung von 4,4'-Di-tert.-butylbenzoylperoxid beschrieben. In DE-B-1 643 599 wird ein Verfahren zur Isolierung und Reinigung von bei Raumtemperatur festen Diacylperoxiden beschrieben. Diese bekannten Verfahren beziehen sich auf die im Vergleich zu Peroxydicarbonaten sehr stabilen Diacylperoxide.

Die Aufgabe der vorliegenden Erfindung besteht nun darin, ein kontinuierlich arbeitendes Verfahren zur Herstellung von insbesondere festen Dialkylperoxydicarbonaten zu schaffen, das eine hohe Ausbeute, bezogen auf den eingesetzten Chlorameisensäureester ermöglicht, das Dialkylperoxydicarbonat mit hoher Reinheit ergibt, eine geringe Umweltbelastung mit sich bringt und ein gleichmäßig und trocken anfallendes Produkt hoher Qualität liefert.

Diese Aufgabe wird nun gelöst durch die kennzeichnenden Merkmale des Hauptanspruchs.

Gegenstand der Erfindung ist daher das Verfahren der eingangs genannten Gattung, das dadurch gekennzeichnet ist, daß man die Umsetzung der Reaktionsteilnehmer in Gegenwart eines Netzmittels unterhalb des Schmelzpunkts des Produkts durchführt und das gebildete Dialkylperoxydicarbonat in geschmolzener Form aus der auf die Schmelztemperatur des Dialkylperoxydicarbonats erhitzten, alkalischen Reaktionsmischung isoliert.

Die Unteransprüche betreffen besonders bevorzugte Ausführungsformen dieses erfindungsgemäßen Verfahrens.

Bei dem beanspruchten Verfahren verwendet man als Ausgangsmaterial einen Chlorameisensäureester der oben definierten allgemeinen Formel II, der vorzugsweise in möglichst reiner Form eingesetzt wird. Bevorzugt setzt man das Chlorformiat mit einer Reinheit von 96 bis 98% und gegebenenfalls mehr ein.

Als weiterer Reaktionspartner wird bei dem erfindungsgemäßen Verfahren Wasserstoffperoxid verwendet, das vorzugsweise in 70prozentiger Form eingesetzt und in einem Überschuß von 10 bis 60% über die stöchiometrisch erforderliche Menge dem Reaktionsgemisch zugeführt wird. Gemäß einer bevorzugten Ausführungsform des erfindungsgemäßen Verfahrens für die Herstellung von Dimyristylperoxydicarbonat bzw. Dicetylperoxydicarbonat verwendet man vorzugsweise einen Wasserstoffperoxidüberschuß von etwa 28% bzw. etwa 54%.

Das Alkalimetallhydroxid wird bei dem erfindungsgemäßen Verfahren in einem Überschuß von 5 bis 30% und vorzugsweise von 5 bis 20% über die stöchiometrisch erforderliche Menge eingesetzt. Man verwendet als Alkalimetallhydroxid vorzugsweise Natriumhydroxid, wenngleich man auch Kaliumhydroxid verwenden kann. Gemäß einer bevorzugten Ausführungsform des beanspruchten Verfahrens setzt man das Alkalimetallhydroxid der Reaktionszone in einer solchen Menge zu, daß die aus der Reaktionszone austretende Reaktionsmischung einen pH-Wert von 10 bis 12,5 und vorzugsweise Von 11 bis 12 aufweist. Dabei führt ein zu hoher pH-Wert zu Ausbeuteverlusten, während ein unterhalb des unteren Grenzwerts liegender pH-Wert höhere Chlorwerte zur Folge hat.

Ein wesentliches Merkmal des erfindungsgemäßen Verfahrens ist darin zu sehen, daß man die Umsetzung in Gegenwart eines Netzmittels bzw. eines oberflächenaktiven Mittels durchführt. Hierfür kann man beliebige, den Reaktionsablauf nicht störend oberflächenaktive Mittel verwenden. Als besonders gut geeignet erwiesen haben sich jedoch anionenaktive Netzmittel, z. B. Sulfobernsteinsäureester und die Natriumalkylpolyglykolätherphosphate bzw. die Gemische aus zwei oder mehreren dieser Netzmittel.

Erfindungsgemäß wird das Netzmittel in einer Menge von 0,8 bis 2,5 g, vorzugsweise 1,1 bis 1,8 g, bezogen auf 100 g des eingesetzten Chlorameisensäureesters, verwendet.

Die erfindungsgemäße Anwendung eines Netzmittels bei der Umsetzung der Reaktionsteilnehmer führt nicht nur zu einem besseren und glatteren Reaktionsverlauf, sondern hat überraschenderweise auch höhere Aktivsauerstoffwerte in dem gebildeten Endprodukt zur Folge.

Vorzugsweise arbeitet man bei einer Reaktionstemperatur von 15 bis 35°C und noch bevorzugter von 20 bis 30°C und führt die Reaktion in der Weise, das heißt unter Anwendung von zwei hintereinander geschalteten Reaktionszonen, daß sich eine Verweilzeit bzw. eine Reaktionszeit von 30 bis 60 und vorzugsweise von 40 bis 50 Minuten ergibt.

Nach der Umsetzung erhält man ein wäßriges Reaktionsgemisch in dem das Dialkylperoxydicarbonat in suspendierter Form vorliegt.

Zur Isolation des Produkts wird das wäßrige, alkalische Reaktionsgemisch möglichst schnell, d. h. in einem möglichst kleinen Reaktionsgefäß bzw. in einem Durchlauferhitzer durch Zugabe von heißem Wasser oder Dampf, auf eine Temperatur erhitzt, die der Schmelztemperatur des Dialkylperoxydicarbonats entspricht. Im Anschluß daran wird geschmolzenes Dialkylperoxydicarbonat unter Anwendung einer kontinuierlich betriebenen Flüssigkeits-Flüssigkeits-Zentrifuge aus der wäßrigen, alkalischen Reaktionsmischung abgetrennt. Mit Vorteil verwendet man hierzu einen Tellerseparator, in dem sich die Phasentrennung sehr schnell und in schonender Weise erreichen läßt.

Es hat sich von Vorteil erwiesen, die erhaltenen Reaktionsmischung oder der gebildeten Dialkylperoxydicarbonatschmelze etwa 0,02 bis 0,1%, vorzugsweise 0,06% Pyridin, bezogen auf das isolierte Dialkylperoxydicarbonat, zuzusetzen, da es hierdurch überraschenderweise gelingt, die statische Aufladung des zu Schuppen verformten Produkts zu verhindern.

Nach der Abtrennung in der Flüssigkeits-Flüssigkeits-Zentrifuge wird die Dialkylperoxydicarbonatschmelze auf einer Kühlwalze zu Schuppen verformt.

Für die Durchführung des erfindungsgemäßen Verfahrens verwendet man als Reaktionsgefäß zweckmäßig einen Rührkessel mit Kühlmantel, wobei die eingesetzten Ausgangsmaterialien über ein Tauchrohr zugesetzt werden können. Bei diesem Tauchrohr handelt es sich vorzugsweise um zwei konzentrisch angeordnete Rohre, wobei über das Innenrohr der Chlorameisensäureester und über das konzentrisch liegende Außenrohr, das etwas länger ist als das Innenrohr, die wäßrigen Komponenten eingeleitet werden.

Mit Hilfe von Zwischenböden kann der Behälterinhalt in zwei oder mehrere Reaktionszonen aufgeteilt werden, wobei in jeder Zone ein Rührpropeller angeordnet ist. Außerdem wurde unter dem Überlaufrohr als Spritzschutz und Rührpulsationsdämpfung eine Trennscheibe vorgesehen.

Da die Umsetzung des Chlorameisensäureesters mit Wasserstoffperoxid sehr träge verläuft und eine Reaktionszeit von 30 bis 60 Minuten erforderlich ist, werden vorzugsweise zwei solche Rührkessel hintereinander geschaltet, wobei auch der zweite Rührkessel ebenso wie der erste ausgerüstet ist. Dabei erfolgt der Zulauf aus dem ersten in das zweite Gefäß über das Tauchrohr in die untere Kammer des zweiten Rührkessels.

Als günstigste Reaktionstemperatur wurden 15 bis 35°C, insbesondere 30°C im ersten Rührkessel und 20 bis 35°C, insbesondere 25°C im zweiten Rührkessel ermittelt. Tiefere Temperaturen ergeben bei der gewählten Verweilzeit höhere Chlorwerte. Eine Verlängerung der Reaktionszeit bzw. der Verweildauer über 60 und insbesondere 50 Minuten hinaus ist unzweckmäßig, da hierdurch die Menge der Reaktionsmischung erhöht wird. Andererseits ist eine höhere Temperatur wegen der geringen Stabilität der alkalischen Wasserstoffperoxidlösung ungeeignet.

Die Isolation der gebildeten Dialkylperoxydicarbonate aus der erhaltenen Reaktionsmischung erfolgt in geschmolzenem Zustand aus der alkalischen Reaktionsmischung sowie sie aus dem zweiten Rührkessel austritt. In geschmolzenem Zustand verhalten sich die festen Dialkylperoxydicarbonate ähnlich wie Fettsäurediacylperoxide, das heißt, sie zersetzen sich sehr rasch. Allerdings sind die Dialkylperoxydicarbonatschmelzen wesentlich instabiler, weshalb zum Beispiel nach dem Abtrennen der Mutterlauge ein Waschen nicht möglich ist. Diese hohe Instabilität der Schmelze erfordert eine möglichst kurze Verweilzeit des Dialkylperoxydicarbonats in geschmolzenem Zustand und ein sehr sorgfältiges Trennen der Schmelze von der Mutterlauge. Dies wird erfindungsgemäß durch die alkalische Trennung unter Verwendung einer Flüssigkeits-Flüssigkeits-Zentrifuge erreicht, wodurch offensichtlich ein Emulsionseffekt auftritt, der die Bildung eines reineren Produkts begünstigt.

Nach dem Verlassen des zweiten Rührgefäßes wird das Reaktionsgemisch in einem kleinen Schmelzkessel (Rührgefäß mit Heizmittel) geführt, in dem durch Zugabe von warmen Wasser und Dampf die Schmelztemperatur des Dialkylperoxydicarbonats (die im Fall des bevorzugt hergestellten Dimyristylperoxydicarbonats bzw. Dicetylperoxydicarbonats 46 bzw. 56°C beträgt) eingestellt. Wegen der kritischen Verweilzeit muß das Gefäß möglichst klein sein. Auch die Anwendung eines Durchlauferhitzers bzw. Mischrohrs ist möglich. Zur Phasentrennung wird die erhaltene Mischung vorzugsweise in einen Tellerseparator gegeben.

Dabei ist die Aufbau des Tellerseparators von Bedeutung, in dem die Anwendung eines Separators mit freiem Austritt der leichten Phase vorteilhaft ist. Zusätzlich sollte das Ablaufblech in der Haube des Separators doppelwandig ausgeführt werden, um so die Schmelze temperieren zu können.

Als Abschluß der Aufarbeitung wird die Percarbonatschmelze auf einer Kühlwalze geschuppt. Das Produkt härtet auf der Walze schnell durch und ergibt Schuppen, deren Größe über die Kühlung der Walze beeinflußt werden kann. Der Staubanteil ist gering. Interessant ist, daß es gemäß einer bevorzugten Ausführungsform der Erfindung möglich ist, die statische Aufladung der Schuppen durch Zugabe von 0,02 bis 0,1%, vorzugsweise 0,06% Pyridin, bezogen auf das isolierte Dialkylperoxydicarbonat, in die Reaktionsmischung zu verhindern.

Das erfindungsgemäße Verfahren besitzt gegenüber den oben angesprochenen Verfahren des Standes der Technik erhebliche Vorteile insofern, als das Verfahren sehr einfach durchzuführen ist, auch bei unreinen Ausgangsmaterialien ein Produkt hoher Reinheit mit hoher Ausbeute liefert und nur sehr wenig organisches Material in das Abwasser gelangen läßt.

Die folgenden Beispiele dienen der weiteren Erläuterung der Erfindung.

### Beispiel 1

Man beschickt ein offenes Rührgefäß, das mit einem Turborührer und Überlaufrohr ausgerüstet ist, pro Minute mit 135 ml Myristylchlorformiat, 65 ml 7,8n-Natriumhydroxidlösung, 11 ml Wasserstoffperoxid (70 Gew.-%), 1,5 ml eines Netzmittelgemisches und 700 ml entmineralisiertem Wasser. Durch Kühlung mit Wasser wird die Reaktionstemperatur bei etwa 30°C gehalten. Die Zugabe der Reaktionsteilnehmer erfolgt durch Tauchrohre, die kurz über dem Kesselboden enden. Das im Überlauf abströmende Reaktionsgemisch wird zur Beendigung der Umsetzung in ein zweites Rührgefäß überführt, wo durch Kühlen eine Temperatur von etwa 25°C aufrechterhalten wird. Die Größe der Rührgefäße ist so gewählt, daß sich eine mittlere Verweilzeit von ca. 50 Minuten ergibt.

Die aus dem zweiten Rührgefäß ablaufende Suspension wird durch Zugabe von etwa 600 ml/min entmineralisiertem Wasser mit einer Temperatur von 80°C auf 46°C gebracht, wodurch das Dimyristylperoxydicarbonat geschmolzen wird. Wichtig ist die möglichst genaue Einhaltung der Temperatur von 46°C, was durch die genau abgemessene Zugabe des Heißwassers erreicht wird. Das Vermischen der Reaktionsuspension mit dem heißen Wasser kann in einem Mischrohr oder möglichst in einem kleinen Rührgefäß geschehen.

In einem nachgeschalteten Tellerseparator wird dann die Dimyristylperoxydicarbonatschmelze von der wäßrigen Phase getrennt. Die aus dem Separator kommende Schmelze wird auf kurzmöglichstem Wege auf eine Kühlwalze geführt, wo sie erstarrt und in Schuppenform abgenommen werden kann.

Man erhält in dieser Weise 116 g/min Dimyristylperoxydicarbonat mit einem Peroxidgehalt von 97%, was einem Gehalt an aktivem Sauerstoff von 3,01% entspricht. Dies entspricht einer Ausbeute von 97,3% der Theorie, bezogen auf das eingesetzte Myristylchlorformiat. Die Analyse des Produkts zeigt einen Chlorgehalt von etwa 0,06%.

### Beispiel 2

Man wiederholt die Verfahrensweise von Beispiel 1, beschickt jedoch das Rührgefäß pro Minute mit 135 ml Cetylchlorformiat, 60 ml 7,8 n-Natriumhydroxidlösung, 12 ml 70gewichtsprozentigem Wasserstoffperoxid, 1,8 ml des Netzmittelgemisches und 930 ml entmineralisiertem Wasser.

Man stellt die Schmelztemperatur durch Zugabe von etwa 1000 ml/min entmineralisiertem Wasser mit einer Temperatur von 90°C auf 56°C ein.

Nach dem Abkühlen des Materials in Schuppenform auf der Kühlwalze erhält man 115 g/min Dicetylperoxydicarbonat mit einem Peroxidgehalt von 97%, entsprechend 2,72% AO, was einer Ausbeute von 97,4% der Theorie, bezogen auf das eingesetzte Chlorformiat, entspricht.

## Patentansprüche

1. Verfahren zur kontinuierlichen Herstellung von Dialkylperoxydicarbonaten der allgemeinen Formel I 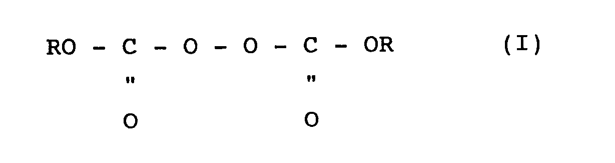 in der R für gleichartige oder verschiedene, geradkettige, cyclische oder verzweigte Alkylgruppen mit 6 bis 18 Kohlenstoffatomen stehen, durch kontinuierliches Umsetzen eines Chlorameisensäureesters der allgemeinen Formel II 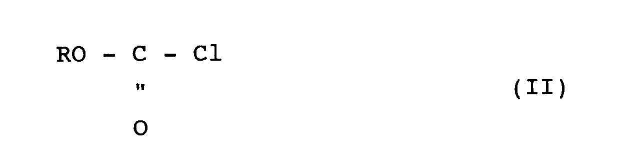 worin R die oben angegebenen Bedeutungen besitzt, in wäßrigem Medium mit Wasserstoffperoxid und einem Alkalimetallhydroxid bei einer Temperatur im Bereich von -10°C bis +50°C in mindestens zwei hintereinander geschalteten Reaktionszonen und kontinuierliches Isolieren des Dialkylperoxydicarbonats aus der lösungsmittelfreien Reaktionsmischung mit Hilfe einer Zentrifuge,
**dadurch gekennzeichnet,**
daß man die Umsetzung in Gegenwart eines Netzmittels unterhalb des Schmelzpunkts des Produkts durchführt und das Dialkylperoxydicarbonat in geschmolzener Form aus der auf die Schmelztemperatur des Dialkylperoxydicarbonats erhitzten alkalischen Reaktionsmischung isoliert.

2. Verfahren nach Anspruch 1, dadurch gekennzeichnet, daß man die Umsetzung unter Anwendung eines Wasserstoffperoxidüberschusses von 10 bis 60% über die stöchiometrisch erforderliche Menge bewirkt.

3. Verfahren nach Anspruch 1, dadurch gekennzeichnet, daß man bei der Umsetzung einen Alkalimetallhydroxidüberschuß von 5 bis 30% über die stöchiometrisch erforderliche Menge anwendet.

4. Verfahren nach Anspruch 3, dadurch gekennzeichnet, daß man das Alkalimetallhydroxid der Reaktionszone in einer solchen Menge zuführt, daß die aus der Reaktionszone austretende Reaktionsmischung pH-Wert von 10 bis 12,5, vorzugsweise 11 bis 12, aufweist.

5. Verfahren nach einem der vorhergehenden Ansprüche, dadurch gekennzeichnet, daß man die Umsetzung unter Anwendung einer Reaktionszeit von 30 bis 60 Minuten durchführt.

6. Verfahren nach einem der vorhergehenden Ansprüche, dadurch gekennzeichnet, daß man anionenaktives Netzmittel, vorzugsweise einen Sulfobernsteinsäureester, ein Natriumalkylpolyglykolätherphosphat oder ein Gemisch aus zwei oder mehreren dieser Netzmittel verwendet.

7. Verfahren nach einem der vorhergehenden Ansprüche, dadurch gekennzeichnet, daß man zur Beschleunigung der Umsetzung den Netzmitteln ein Di-tert.-alkylacetylenglykol zusetzt.

8. Verfahren nach einem der vorhergehenden Ansprüche, dadurch gekennzeichnet, daß man das Netzmittel in einer Menge von 0,8 bis 2,5 g Netzmittel pro 100 g eingesetzten Chlorameisensäureesters verwendet.

9. Verfahren nach einem der vorhergehenden Ansprüche, dadurch gekennzeichnet, daß man die Isolation des Dialkylperoxydicarbonats mit Hilfe einer kontinuierlich betriebenen Flüssigkeits-Flüssigkeits-Zentrifuge bewirkt.

10. Verfahren nach Anspruch 9, dadurch gekennzeichnet, daß man als Flüssigkeits-Flüssigkeits-Zentrifuge einen Tellerseparator verwendet.

11. Verfahren nach einem der vorhergehenden Ansprüche, dadurch gekennzeichnet, daß man der Reaktionsmischung oder der Dialkylperoxydicarbonatschmelze etwa 0,02 bis 0,1% Pyridin, bezogen auf das isolierte Dialkylperoxydicarbonat, zusetzt.

12. Verfahren nach einem der vorhergehenden Ansprüche, dadurch gekennzeichnet, daß man die abgetrennte Dialkylperoxydicarbonatschmelze auf einer Kühlwalze zu Schuppen verformt.

## Claims

1. Process for the continuous production of dialkylperoxydicarbonates of the general formula I 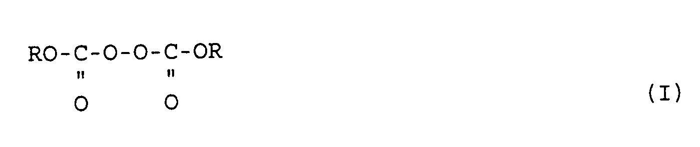 in which R stands for the same or different, straight-chained, cyclic or branched alkyl groups with 6 to 18 carbon atoms, by the continuous reaction of a chloroformic acid ester of the general formula II 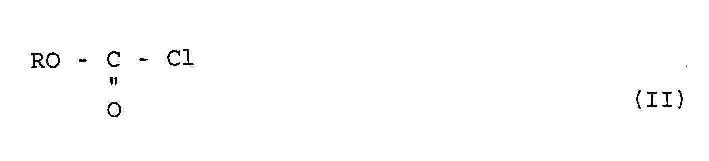 wherein R has the above-given meanings, in an aqueous medium with hydrogen peroxide and an alkali metal hydroxide at a temperature in the range of -10°C to +50°C in at least two reaction zones connected one behind the other and continuous isolation of the dialkylperoxydicarbonate from the solvent-free reaction mixture with the help of a centrifuge, **characterised in that** one carries out the reaction in the presence of a wetting agent below the melting point of the product and isolates the dialkylperoxydicarbonate in molten form from the alkaline reaction mixture heated to the melting temperature of the dialkylperoxydicarbonate.

2. Process according to claim 1, **characterised in that** one brings about the reaction with the use of a hydrogen peroxide excess of 10 to 60 % over the stoichiometrically necessary amount.

3. Process according to claim 1, **characterised in that**, in the reaction, one uses an alkali metal hydroxide excess of 5 to 30 % over the stoichiometrically necessary amount.

4. Process according to claim 3, **characterised in that** one supplies the alkali metal hydroxide to the reaction zone in such an amount that the reaction mixture emerging from the reaction zone has a pH value of 10 to 12.5, preferably of 11 to 12.

5. Process according to one of the preceding claims, **characterised in that** one carries out the reaction with the use of a reaction time of 30 to 60 minutes.

6. Process according to one of the preceding claims, **characterised in that** one uses an anion-active wetting agent, preferably a sulphosuccinic acid ester, a sodium alkylpolyglycol ether phosphate or a mixture of two or more of these wetting agents.

7. Process according to one of the preceding claims, **characterised in that**, for the acceleration of the reaction, one adds a di-tert.-alkylacetyleneglycol to the wetting agent.

8. Process according to one of the preceding claims, **characterised in that** one uses the wetting agent in an amount of 0.8 to 2.5 g of wetting agent per 100 g of chloroformic acid ester used.

9. Process according to one of the preceding claims, **characterised in that** one brings about the isolation of the dialkylperoxydicarbonate with the use of a continuously operated liquid-liquid centrifuge.

10. Process according to claim 9, **characterised in that**, as a liquid-liquid centrifuge, one uses a disc separator.

11. Process according to one of the preceding claims, **characterised in that** one adds about 0.02 to 0.1 % of pyridine, referred to the isolated dialkylperoxydicarbonate, to the reaction mixture or to the dialkylperoxydicarbonate melt.

12. Process according to one of the preceding claims, **characterised in that** one shapes the separated dialkylperoxydicarbonate melt into flakes on a cooling roller.

## Revendications

1. Procédé pour la préparation en continu de dialcoyl peroxydicarbonates représentés par la formule générale (I) : 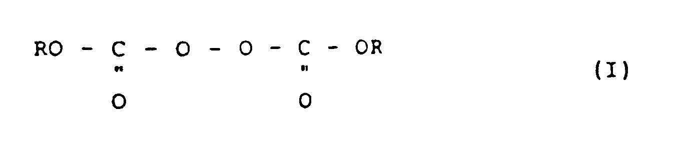 dans laquelle R signifie des groupes alcoyles identiques ou différents, à chaîne linéaire, cyclique ou ramifié avec 6 à 18 atomes de carbone, par la mise en réaction en continu d'un chloroformiate représenté par la formule générale (II): 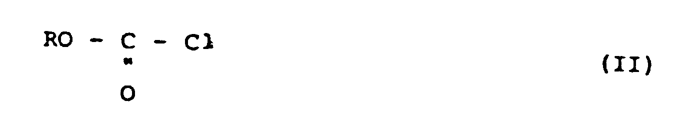 où R possède les significations indiquées ci-dessus, dans un milieu aqueux avec du peroxyde d'hydrogène et un hydroxyde de métal alcalin, à une température dans la plage de -10 à +50°C, dans au moins deux zones réactionnelles disposées en série et l'isolation continue du dialcoyl peroxydicarbonate à partir du mélange réactionnel sans solvant à l'aide d'une centrifugeuse, caractérisé en ce que la réaction s'effectue en présence d'un agent tensio-actif au-dessous du point de fusion du produit et en ce que l'on isole le dialcoyl peroxydicarbonate sous forme fondue à partir du mélange réactionnel alcalin chauffé à la température de fusion du dialcoyl peroxydicarbonate.

2. Procédé selon la revendication 1, caractérisé en ce qu'on effectue la réaction en utilisant un excès de peroxyde d'hydrogène de 10 à 60% par rapport à la quantité stoechiométrique nécessaire.

3. Procédé selon la revendication 1 caractérisé en ce qu'on utilise pour la réaction, un excès d'hydroxyde de métal alcalin de 5 à 30% par rapport à la quantité stoechiométrique nécessaire.

4. Procédé selon la revendication 3, caractérisé en ce qu'on introduit l'hydroxyde de métal alcalin dans la zone de réaction en une quantité telle que le mélange de réaction sortant de la zone de réaction présente un pH de 10 à 12,5, de préférence de 11 à 12.

5. Procédé selon l'une des revendications précédentes, caractérisé en ce qu'on effectue la réaction en appliquant un temps de réaction de 30 à 60 minutes.

6. Procédé selon l'une des revendications précédentes, caractérisé en ce qu'on utilise un agent tensio-actif anionique, de préférence un sulfosuccinate, un alcoylpolyglycoléther phosphate de sodium ou un mélange de deux ou plusieurs de ces agents tensio-actifs.

7. Procédé selon l'une des revendications précédentes, caractérisé en ce qu'on ajoute un di-ter-alcoylacétylène glycol pour accélérer la réaction, aux agents tensio-actifs.

8. Procédé selon l'une des revendications précédentes, caractérisé en ce qu'on utilise l'agent tensio-actif en une quantité de 0,8 à 2,5 g d'agent pour 100 g de chloroformiate mis en jeu.

9. Procédé selon l'une des revendications précédentes, caractérisé en ce qu'on réalise l'isolation du dialcoyl peroxydicarbonate à l'aide d'une centrifugeuse liquide-liquide fonctionnant en continu.

10. Procédé selon la revendication 9, caractérisé en ce qu'à titre de centrifugeuse liquide-liquide, on utilise un séparateur à plateaux.

11. Procédé selon l'une des revendications précédentes, caractérisé en ce qu'on ajoute au mélange réactionnel ou au dialcoyl peroxydicarbonate fondu, environ 0,02 à 0,1% de pyridine, par rapport au dialcoyl peroxydicarbonate isolé.

12. Procédé selon l'une des revendications précédentes, caractérisé en ce qu'on met la masse fondue de dialcoyl peroxydicarbonate séparée, sous forme d'écailles, sur un cylindre de refroidissement.
